# FASCICULE DE BREVET EUROPEEN

(11) **EP 2 791 312 B1**
(45) Date de publication et mention de la délivrance du brevet: **12.05.2021**
(21) Numéro de dépôt: 12813909.4
(22) Date de dépôt: 12.12.2012
(51) Int. Cl.: C12M 1/107, C12M 1/00, C12M 3/00, C12M 1/34, C12M 1/36

(54) **INSTALLATION DE MÉTHANISATION MODULAIRE DE MATIÈRES ORGANIQUES SOLIDES, COMPOSÉE D'UN NOMBRE VARIABLE DE MODULES DE DIGESTION TRANSPORTABLES, ET PROCÉDÉ DE COMMANDE D'UNE TELLE INSTALLATION**
MODULARE METHANISIERUNGSANLAGE FÜR ORGANISCHEN FESTSTOFF AUS EINER VARIABLEN ANZAHL VON TRANSPORTIERBAREN VERDAUUNGSMODULEN UND VERFAHREN ZUR STEUERUNG EINER SOLCHEN ANLAGE
MODULAR METHANISATION FACILITY FOR SOLID ORGANIC MATTER, COMPOSED OF A VARIABLE NUMBER OF TRANSPORTABLE DIGESTION MODULES, AND METHOD FOR CONTROLLING SUCH A FACILITY

(30) Priorité: 12.12.2011 FR 1103803
(43) Date de publication de la demande: 22.10.2014
(73) Titulaire: UNIVERSITE DE TECHNOLOGIE DE COMPIEGNE, 60200 Compiègne (FR)
(72) Inventeur: LESPINARD, Olivier, F-92250 La Garenne-colombes (FR); NONUS, Maurice, F-60150 Villers S/coudun (FR); PAUSS, André, F-60200 Compiegne (FR); RIBEIRO, Thierry, F-80250 Ailly S/noye (FR)
(74) Mandataire: Delorme, Nicolas
(86) Numéro de dépôt international: PCT/FR2012/052903
(87) Numéro de publication internationale: WO 2013/088067

(56) Documents cités:
- EP-A1- 2 251 408
- DE-A1- 2 934 680
- DE-A1- 3 526 820
- DE-A1- 4 315 603
- DE-A1- 4 409 487
- DE-A1-102009 025 329
- DE-U1-202005 012 340
- DE-U1-202006 002 757
- DE-U1-202010 000 437
- FR-A- 1 094 014
- FR-A- 2 374 413
- FR-A- 2 617 130
- NL-A- 7 608 061
- US-A- 1 924 143
- US-A- 3 189 376
- US-A- 5 552 038
- US-A1- 2011 039 315

## Description

La présente demande de brevet se rapporte à une installation modulaire et évolutive de méthanisation de déchets organiques, notamment d'origine agricole, à un digesteur mobile pour une telle installation, et à un procédé de commande d'une telle installation composée d'un ou plusieurs modules.

On connaît de longue date le procédé de fermentation anaérobie des matières organiques (aussi appelés substrats), permettant d'une part de produire un biogaz riche en méthane et d'autre part de transformer ces déchets en amendement pour les terres agricoles qui s'apparente à un compost.

Par exemple, le document DE202010000437 concerne une installation de biogaz transportable et modulaire avec une chambre de fermentation formée par au moins deux modules de fermentation transportables qui peuvent être connectés ou reliés entre eux sur leurs faces d'extrémité ; le document DE4409487 concerne un procédé de fermentation de déchets bruts organiques à l'aide d'une pluralité de bioréacteurs qui libèrent de l'eau de percolation et qui sont chargés successivement avec les déchets bruts ; le document DE202006002757 concerne un bioréacteur pour la méthanisation de la biomasse à haute teneur en solides, avec un digesteur verrouillable étanche au gaz, une décharge de biogaz, une ouverture de chargement et de déchargement pour remplir et vider le digesteur avec de la biomasse ; le document DE 102009025329 concerne également un procédé d'échange de biogaz entre un ou plusieurs bioréacteurs ; le document FR10941014 concerne un procédé et appareil pour la production du gaz de fumier ; le document DE202005012340 concerne une installation de biogaz avec au moins un fermenteur et des modules qui hébergent des parties techniques de l'installation, en particulier la technologie de contrôle et de régulation, la technologie de la pompe et au moins une centrale thermique et électrique combinée et le document DE4315603 concerne un cadre tubulaire avec entretoises tubulaires s'étendant horizontalement et verticalement, qui sont connectées de manière amovible les unes aux autres aux points d'intersection via un élément de connexion.

Dans tous les cas, il importe de pouvoir collecter le biogaz produit afin de le valoriser. En effet le méthane issu de cette fermentation, peut faire fonctionner des moteurs de cogénération (production d'électricité et de chaleur) ou des équipements thermiques, chaudières turbines, etc. pour chauffer des installations domestiques ou industrielles, etc.

Selon la nature des matières organiques, on recourt soit à des procédés « en voie liquide » qui opèrent à des taux de dilution élevés dans de l'eau, soit à des procédés en « voie sèche » ou « voie solide ».

Les installations de méthanisation des déchets solides, que l'on trouve plus particulièrement dans les exploitations d'élevage sur litière paillées comportent de grandes fosses bétonnées enterrées que l'on charge de matières organiques, puis que l'on couvre de bâches afin de permettre la réaction de fermentation anaérobie. Dans d'autres cas, on utilise des cellules bétonnées munies d'une porte hermétique.

Dans certains cas, on envisage l'installation d'une pompe à percolât, permettant de faire remonter le liquide chargé en bactéries qui se dépose au fond de la fosse et de le pulvériser sur le dessus du chargement de la fosse.

On attend jusqu'à deux mois pour que la fermentation se déroule correctement, puis l'on vient prendre le résidu issu de la digestion (appelé de 30 manière générique « digestât » dans ce qui suit) que l'on charge dans une benne au moyen d'un tracteur, en vue de l'épandre sur les champs.

Cette manière de faire présente de nombreux inconvénients. Tout d'abord, la réalisation de grandes fosses ou de cellules bétonnées est coûteuse, nécessite un permis de construire et donne lieu à des taxes fiscales liées au caractère immobilier d'un tel ouvrage.

De plus, le volume d'une telle fosse ou cellule est important. Il faut donc un certain temps pour accumuler les substrats nécessaires pour la remplir en une fois. Pendant ce temps de stockage, les substrats se dégradent et perdent une part de leur potentiel de production de biogaz et sont fréquemment sources de nuisances (odeurs, insectes...).

Ceci peut être très gênant par exemple si l'on souhaite apporter des substrats dans la fosse, alors que la réaction de méthanisation est lancée, ou bien au contraire si l'on souhaite récupérer du digestât en vue de son épandage, alors qu'on est en plein milieu de cycle.

De plus, en cas de panne de la réaction de fermentation, comme cela arrive parfois, le volume de déchets à évacuer et retraiter est très important.

La présente invention a ainsi notamment pour but de fournir une installation de méthanisation de substrats organiques et notamment de déchets d'origine agricole qui ne présente pas ces inconvénients.

En particulier, la présente invention a notamment pour but de fournir une installation de méthanisation des matières organiques qui soit modulaire, flexible, et qui permette de récupérer de manière très simple les gaz issus de la fermentation de ces déchets.

On atteint ce but de l'invention notamment avec un module digesteur comprenant un conteneur mobile de méthanisation, comprenant un réceptacle de réception de matières organiques telles que déchets d'origine agricole, un couvercle étanche, une porte latérale étanche, et un connecteur de récupération des gaz de fermentation, le réceptacle, le couvercle et la porte étant hermétiques et isolés thermiquement, ledit réceptacle comprenant une pluralité de capteurs, choisis dans le groupe comprenant des capteurs de pression, des capteurs de température et des capteurs de niveau de liquide, et des moyens de connexion disposés à l'extérieur dudit réceptacle, pour relier ces capteurs à un circuit électrique, lesdits moyens de connexion étant groupés dans une même zone dudit conteneur, facilement accessible.

Suivant d'autres caractéristiques optionnelles de ce module digesteur :
- ledit conteneur est conformé pour être placé sur un camion ou une remorque agricole par exemple de type « ampliroll » ;
- ledit couvercle est rigide ;
- ledit couvercle est muni de manchons de préhension ;
- des verrous à genouillère sont disposés entre ledit réceptacle et ledit couvercle pour maintenir ce dernier en compensant la pression exercée par le biogaz et assurer une parfaite étanchéité au niveau du joint entre le couvercle et le bord du réceptacle ;
- ledit couvercle peut comprendre un cadre rigide sur lequel est tendu au moins une bâche souple ;
- alternativement, deux bâches souples sont tendues sur ledit cadre rigide, un isolant thermique étant disposé entre ces deux bâches souples ;
- ledit réceptacle comprend un circuit de diffusion de percolât, comprenant un connecteur extérieur d'entrée de percolât, une pluralité de diffuseurs de percolât disposés dans la partie supérieure du réceptacle, en communication de fluide avec ledit connecteur d'entrée du percolât, et un connecteur de sortie du percolât, en communication de fluide avec une zone de récupération du percolât ayant traversé lesdits déchets agricoles (substrats) ;
- ladite zone de récupération du percolât comprend un fond drainant du réceptacle, et une chambre de soutirage du percolât, séparée du reste du volume dudit réceptacle par une paroi ajourée ;
- ledit réceptacle comprend un circuit de chauffage à fluide caloporteur, comprenant des connecteurs extérieurs d'entrée et de sortie du fluide caloporteur, et un serpentin s'étendant entre ces deux connecteurs, à l'intérieur dudit réceptacle ;
- ledit réceptacle comprend un connecteur d'entrée d'air de purge sous pression.

La présente invention se rapporte également à une station d'accueil pour module digesteur conforme à ce qui précède, comprenant des connecteurs complémentaires de ceux dudit conteneur, et aptes à être reliés à des circuits respectifs de récupération des gaz de fermentation, de percolât, de fluide caloporteur, de transmission de données et de courants électriques.

La présente invention se rapporte également à une installation de méthanisation de matières organiques telles que des déchets d'origine agricole, comprenant :
- une pluralité de modules digesteurs et de stations d'accueil conformes à ce qui précède, dont les connecteurs complémentaires et moyens de connexion respectifs sont aptes à être accouplés en début de réaction de fermentation, et à être découplés en fin de réaction,
- un circuit de récupération des gaz de fermentation, relié d'une part aux connecteurs complémentaires de récupération des gaz des stations d'accueil, et d'autre part à au moins une unité stockage et une unité de traitement,
- des circuits d'alimentation et de collecte des fluides et données et courants électriques reliant les stations d'accueil.

Grâce à ces caractéristiques, la méthanisation des matières organiques peut s'effectuer dans des conteneurs qui par nature sont modulaires, et dont le nombre peut être choisi en fonction des besoins.

On peut remplir de déchets chaque conteneur de manière décalée dans le temps, ce qui d'une part rend au moins une partie de ces conteneurs disponible en permanence pour l'apport de nouvelle matière organique (substrats), et d'autre part permet de récupérer de manière périodique du digestât, en vue de son épandage.

La mise en route des réactions de méthanisation peut être décalée dans le temps pour adapter les traitements à la disponibilité des substrats ou à l'alimentation optimale des équipements de valorisation du biogaz.

Suivant d'autres caractéristiques optionnelles de cette installation :
- ladite unité de traitement est choisie dans le groupe comprenant une unité de stockage, une unité de filtration ou d'épuration du biogaz, une unité de cogénération, une unité de brûlage, une unité de compression, une unité de distribution vers le réseau de gaz de ville, une unité de chargement de véhicule utilisant le biogaz comme carburant : ainsi, selon les possibilités de chaque exploitation agricole, un usage optimal des gaz de fermentation pourra être envisagé ;
- cette installation comprend un circuit de distribution du percolât, comprenant au moins une cuve de stockage de percolât, au moins une pompe et une pluralité d'électrovannes interposées entre cette pompe et chaque connecteur d'entrée et de sortie du percolât de chaque station d'accueil ;
- cette installation comprend un réseau de chaleur, composé d'un circuit de circulation de fluide caloporteur, comprenant au moins une source de chaleur et une pluralité de vannes ou de circulateurs interposés entre cette source de chaleur et chaque connecteur d'entrée et de sortie du fluide caloporteur de chaque station d'accueil ;
- cette installation comprend un circuit de collecte du biogaz, comprenant au moins un réservoir de stockage et une pluralité d'électrovannes interposées entre les équipements de valorisation du biogaz et chaque connecteur de sortie du biogaz de chaque station d'accueil ;
- lesdites stations d'accueil et lesdits circuits forment des modules disponibles en kit de montage ;
- lesdits circuits sont enterrés ou non, protégés ou non dans des goulottes ou caniveaux techniques.

La présente invention se rapporte également à un procédé de commande d'une installation conforme à ce qui précède, dans lequel on analyse le déroulement de la réaction de fermentation dans chaque conteneur mobile à partir des données transmises par lesdits capteurs, et on adapte à partir de cette analyse certains réglages de ladite installation de manière à optimiser la réaction de fermentation.

Suivant d'autres caractéristiques optionnelles de ce procédé :
- les circuits de fluides reliant les conteneurs peuvent être connectés en parallèle comme représenté dans les figures jointes, ou en série ;
- le biogaz collecté peut être réinjecté dans un conteneur de manière à prévenir la décantation du percolât ;
- les réglages que l'on adapte sont choisis dans le groupe comprenant la pression des gaz de fermentation, la nature du percolât stocké en cuve, le mélange des percolâts provenant des différents conteneurs mobiles, la température du fluide caloporteur, les apports de divers produits permettant de stimuler ou réguler la réaction de dégradation biologique ;
- on constitue des bases de données permettant de connaître les paramètres de réglage nécessaires à l'optimisation des réactions de fermentation en fonction des caractéristiques des matières à traiter.

D'autres caractéristiques et avantages de la présente invention apparaîtront à la lumière de la description qui va suivre, et à l'examen des figures ci-annexées, dans lesquelles :
- la figure 1 est une vue en perspective d'une installation selon l'invention,
- la figure 2 est une vue schématique de dessus de l'installation de la figure 1,
- la figure 3 est une vue en perspective d'un conteneur mobile de méthanisation selon l'invention, faisant partie de l'installation des figures 1 et 2,
- la figure 4a est une vue de l'avant du conteneur mobile de la figure 3,
- la figure 4b est une vue de l'arrière de ce conteneur mobile,
- la figure 5 est une vue schématique des organes intérieurs du conteneur mobile des figures 3, 4a, 4b, présentant notamment les circuits de chauffage et de percolation,
- la figure 6 est une vue schématique de la zone de liaison d'un couvercle souple avec un réceptacle, l'ensemble formant une variante de réalisation du conteneur mobile des figures 3 à 5,
- la figure 7 est une vue schématique des circuits hydrauliques de l'installation des figures 1 et 2,
- la figure 8 est une vue schématique des circuits électriques de cette installation, et
- la figure 9 est une vue schématique du circuit de récupération des gaz de fermentation de cette installation.

Sur l'ensemble de ces figures, des références identiques ou analogues désignent des organes ou ensembles d'organes identiques ou analogues.

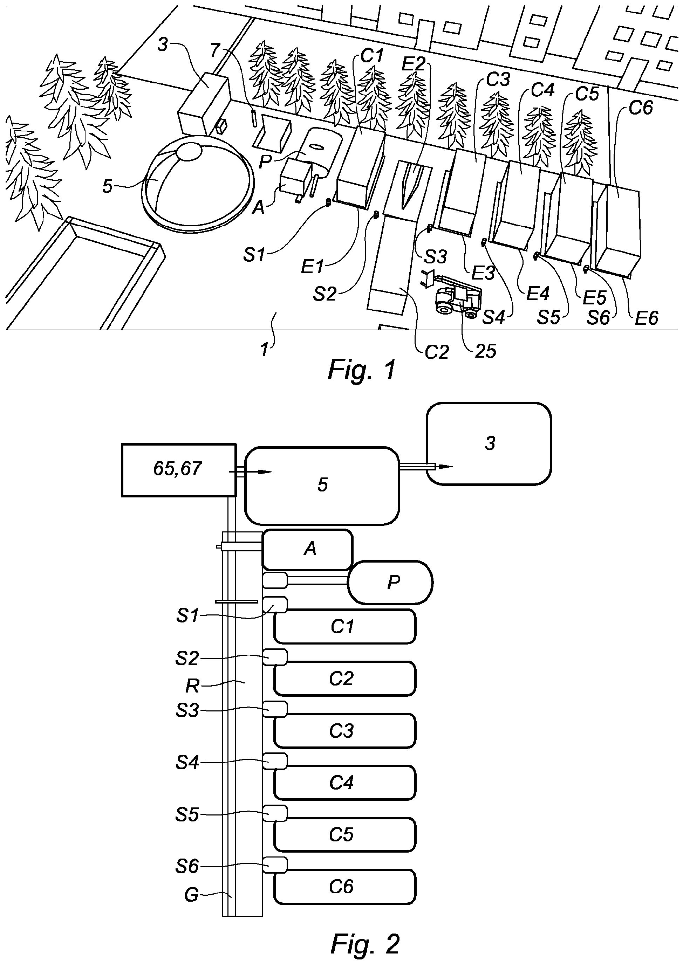

On se reporte à présent aux figures 1 et 2, sur lesquelles on peut voir une installation selon l'invention comportant 6 modules de digestion. Ce nombre n'est nullement limitatif.

Une telle installation est particulièrement destinée aux exploitations agricoles produisant des déchets verts ou des effluents d'élevage, mais cela n'est bien entendu nullement limitatif.

L'installation des figures 1 et 2 repose sur une plateforme stabilisée 1, pouvant être formée d'un sol compacté ou d'une dalle en béton, présentant une pente suffisante pour permettre l'écoulement des eaux de pluie.

Sur cette plateforme se trouvent des emplacements E1, ...E6, destinés à recevoir des conteneurs mobiles de méthanisation C1, ...C6, qui seront détaillés par la suite.

En face de chaque emplacement E1, ...E6, se trouve une borne ou station d'accueil respective S1, S6, dont il sera question plus loin.

Les stations d'accueil S1, ...S6 sont reliées à des circuits hydrauliques et électriques R, disposés de préférence sous la plateforme 1, et communiquant respectivement avec une armoire de commande A et une cuve de percolât P, étant noté que dans le cadre de la présente invention, le terme « percolât » désigne un liquide fortement chargé en bactéries adapté pour réaliser la fermentation anaérobie des déchets d'origine agricole.

Sous le niveau du sol, et de l'autre côté des emplacements E1, ...E6 par rapport aux circuits R, se trouve un circuit de récupération des gaz de 5 fermentation G, relié à une unité de cogénération 3, à un gazomètre 5 et à une torchère 7.

On rappelle ici qu'une unité de cogénération 3 consiste en un moteur apte à engendrer du courant électrique lorsqu'il est alimenté en gaz, et notamment en biogaz pouvant provenir de la fermentation anaérobie de déchets organiques, à savoir essentiellement du méthane.
Le gazomètre 5, pouvant se présenter sous la forme d'une membrane souple étanche au gaz, par exemple enduite de PVC, ou tout autre type de gazomètre, constitue un réservoir de stockage des gaz de fermentation.

Un équipement de traitement de H2S gazeux peut y être adjoint.

La torchère 7 constitue, quant à elle, un moyen de brûlage des gaz de fermentation, lorsqu'aucune autre utilisation de ces gaz n'est souhaitable ou possible.

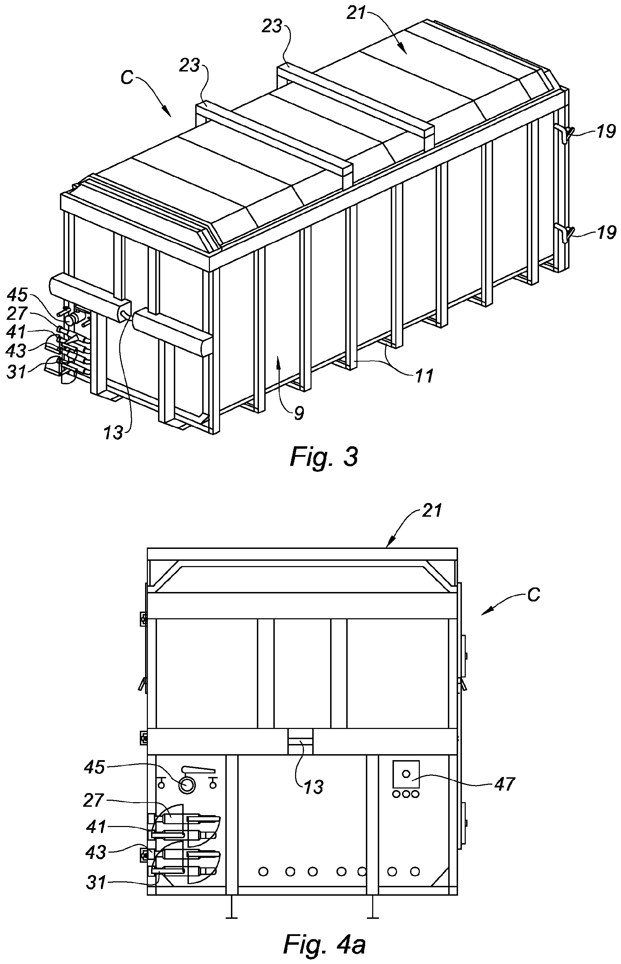

On se reporte à présent aux figures 3 à 6, sur lesquelles on peut voir de manière détaillée la structure d'un conteneur mobile de méthanisation C selon l'invention.

Un tel conteneur C comporte un réceptacle 9 de forme sensiblement parallélépipédique, analogue aux bennes à déchets de la technique antérieure ou tout autre système de bennes ou containers amovibles.

Les armatures 11 permettent de renforcer ce réceptacle 9, et une barre de préhension 13 située sur la face avant de ce réceptacle 9 permet sa préhension par un crochet hydraulique.

Un tel système de crochet hydraulique connu sous le nom d'« Ampliroll » se trouve notamment sur des camions ou remorques : sur ces remorques munies de rouleaux, le crochet hydraulique permet de charger ou décharger le conteneur C par un simple mouvement de traction ou de poussée du crochet.

Sur sa face arrière, c'est-à-dire opposée à la face sur laquelle se trouve la barre de traction 13, le réceptacle 9 comporte une porte 15 à ouverture latérale, c'est-à-dire une porte apte à pivoter autour d'un axe sensiblement vertical Z grâce à des charnières 17 disposées sur le côté du réceptacle 9.

Un système de verrouillage approprié pouvant comprendre par exemple des crochets 19 montés pivotant sur le réceptacle 9 et aptes à coopérer avec des ergots correspondants solidaires de la porte 15, permet une fermeture sécurisée de cette porte.

Plus précisément, il convient de noter que cette fermeture doit être étanche, de manière à pouvoir contenir les gaz sous pression qui se trouvent à l'intérieur du réceptacle 9.

A cet effet, un joint d'étanchéité est prévu entre le réceptacle 9 et la porte 15, lequel joint doit être comprimé par le dispositif de fermeture et de verrouillage 19, de manière à réaliser la fonction d'étanchéité.

Le réceptacle 9 est fermé dans sa partie supérieure par un couvercle amovible 21, muni de deux manchons de préhension 23, aptes à être soulevés par la fourche d'un tracteur agricole, ou par un engin à bras télescopique 25 tel que celui visible sur la figure 1, ou par tout autre dispositif de levage.

Il faut comprendre que l'ouverture du couvercle 21 se fait par simple soulèvement de celui-ci, sans qu'aucune articulation ne soit nécessaire.

Dans une autre variante possible mais non représentée, le couvercle 21 pourrait être monté articulé sur le réceptacle 9.

Pour réaliser la fonction d'étanchéité, il est prévu un joint entre le bord supérieur du réceptacle 9 et le couvercle 21, lequel joint est comprimé sous l'effet du poids de ce couvercle et de la traction des verrous.

De manière à garantir qu'aucune fuite de gaz ne puisse avoir lieu, on prévoit des verrous à genouillère (appelées aussi « sauterelles ») entre le bord supérieur du réceptacle 9 et le couvercle 21, sur toute la périphérie de celui-ci.

De préférence, l'un de ces verrous à genouillère est taré à une pression limite, de sorte que si la pression à l'intérieur du réceptacle 9 dépasse ce seuil, ce verrou saute et puisse ainsi libérer du gaz : on réalise de la sorte une fonction de soupape de sécurité.

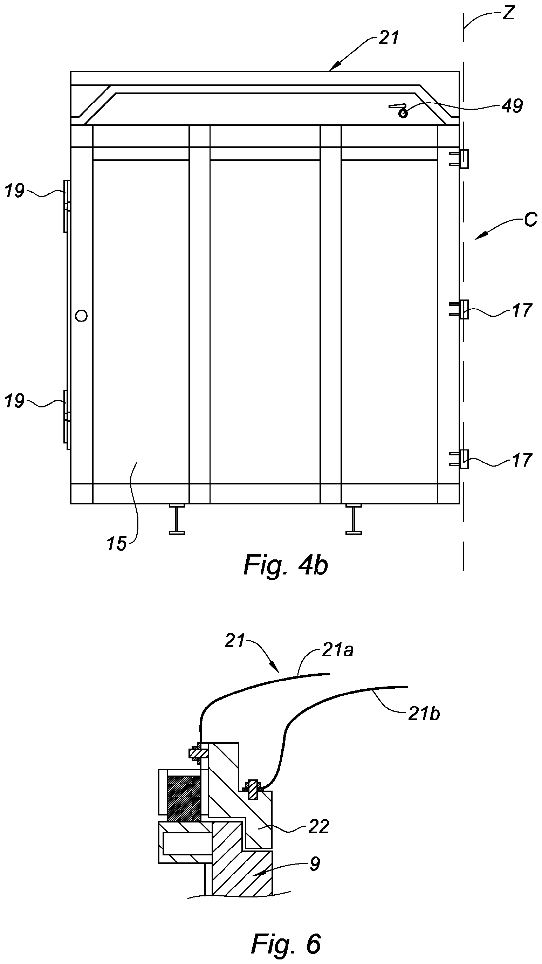

De manière alternative, et comme représenté à la figure 6, le couvercle 21 pourrait être formé d'une membrane souple 21a, fixée sur un 35 cadre rigide 22, lui-même posé et maintenu sur le bord supérieur du réceptacle 9.

Cette membrane 21a pourrait elle-même être doublée d'une membrane intérieure 21b, de manière que l'espace situé entre ces deux membranes puisse être rempli d'air ou d'un autre gaz permettant d'obtenir une bonne isolation thermique de l'intérieur du réceptacle 9 vis-à-vis de l'extérieur.

S'agissant justement de l'isolation thermique, il s'agit ici d'un point technique important vis-à-vis de l'efficacité des réactions de fermentation qui doivent avoir lieu à l'intérieur du conteneur mobile C : toutes les parois du réceptacle 9 ainsi que le couvercle 21 doivent en effet être isolés thermiquement, par exemple avec de la laine de roche ou tout autre matériau isolant plus performant, de manière que la température à l'intérieur du conteneur C puisse se stabiliser à des températures souhaitées notamment autour de 37° Celsius, (procédés mésophiles) ou 55°C (procédés thermophiles) températures optimales de développement des bactéries réalisant la décomposition anaérobie des matières organiques.

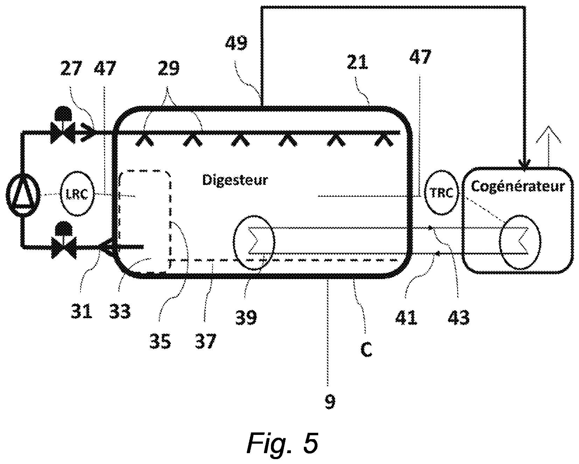

On se reporte à présent à la figure 5, sur laquelle on peut voir que le conteneur C comporte un circuit de diffusion de percolât, comprenant un connecteur extérieur d'entrée de percolât 27 relié à une pluralité de diffuseurs de percolât 29 disposés dans la partie supérieure du réceptacle 9, ainsi qu'un connecteur de sortie du percolât 31, en communication avec une chambre de soutirage 33 du percolât, séparée du reste du volume du réceptacle 9 par une paroi ajourée 35, et communiquant avec un fond drainant 37 du réceptacle.

A noter qu'un tel fond drainant permet l'écoulement par gravité du liquide provenant des déchets situés à l'intérieur de ce réceptacle.
A noter également que les diffuseurs de percolât 29 peuvent être montés sous le couvercle 21 : il faut dans ce cas prévoir des moyens permettant de déconnecter de manière simple ces diffuseurs du reste du circuit lorsqu'on soulève le couvercle 21.

Une variante non représentée, permettant d'une part d'alléger le couvercle 21, et d'autre part de supprimer le problème de la déconnexion des diffuseurs de percolât du reste du circuit lorsqu'on soulève le couvercle 21, consiste à monter ces diffuseurs à l'intérieur des parois du réceptacle 9, à proximité des bords supérieurs de ce réceptacle.

Le conteneur C comporte par ailleurs un circuit de chauffage, consistant essentiellement en un serpentin 39 s'étendant à l'intérieur du réceptacle 9, et débouchant à l'extérieur de celui-ci par des connecteurs d'entrée 41 et de sortie 43 d'un fluide caloporteur tel que l'eau, l'eau glycolée ou tout autre liquide plus performant.

Sur sa face avant, le conteneur C comprend également un connecteur d'entrée d'air de purge sous pression 45, dont le rôle va être expliqué par la suite.

La face avant du conteneur mobile C est également pourvue d'une série de connecteurs électriques 47, reliés à des capteurs tels que des capteurs de température, de niveau de liquide et de pression, disposés à l'intérieur du réceptacle 9.

Sur sa face arrière, le conteneur C comporte un connecteur de récupération des gaz de fermentation 49.

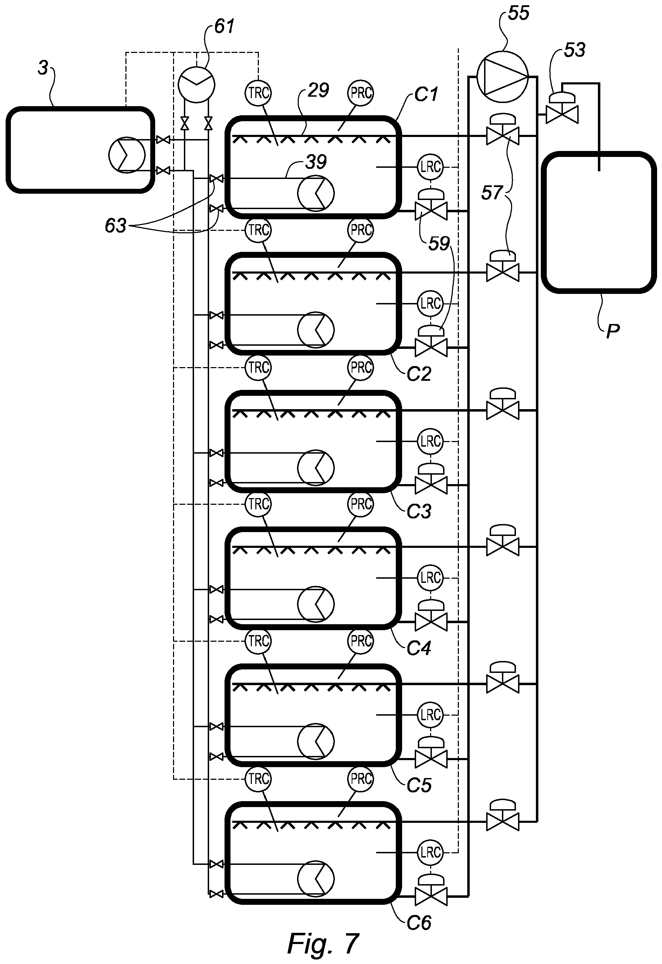

On se reporte à présent à la figure 7, sur laquelle on peut voir des circuits hydrauliques de l'installation selon l'invention ; sur cette figure et sur les suivantes, TRC désigne un capteur de température, PRC désigne un capteur de pression, LRC désigne un capteur de niveau de liquide.

Sur cette figure, il faut comprendre que les circuits situés à l'extérieur des conteneurs C1, C6 correspondent aux circuits enterrés R de la figure 1, et sont donc installés à résidence, tandis que les conteneurs C1, ...C6 sont mobiles.

La connexion des circuits hydrauliques et électriques des conteneurs C1, ...C6 aux réseaux hydrauliques et électriques qui demeurent à résidence, s'effectue par l'intermédiaire des connecteurs 27, 31, 41, 43, 45, 47 représentés à la figure 4a, destinés à être reliés à des connecteurs correspondants situés sur les stations d'accueil respectives S (voir figure 1).

Comme on peut le voir sur la figure 7, la partie fixe de l'installation comporte une cuve de stockage de percolât P, communiquant par l'intermédiaire d'une électrovanne 53, avec un circuit comprenant une unique pompe 55 et une pluralité d'électrovannes 57 et 59 interposées ente cette pompe et chaque connecteur d'entrée 27 et de sortie 31 de chaque conteneur C, permettant ainsi d'alimenter en percolât les diffuseurs 29 de chacun des conteneurs C, et de récupérer du percolât au fond des réceptacles 9.

Le circuit hydraulique comprend également un circuit de circulation d'un fluide caloporteur tel que de l'eau chaude, la chaleur étant fournie par au moins une source de chaleur telle que le cogénérateur 3 ou tout autre système de chaudière 61.

Des vannes manuelles 63 (ou éventuellement des électrovannes ou encore des circulateurs électriques) permettent de réguler le débit de fluide caloporteur circulant entre ces sources de chaleur et chaque serpentin 39 disposé à l'intérieur des conteneurs C.

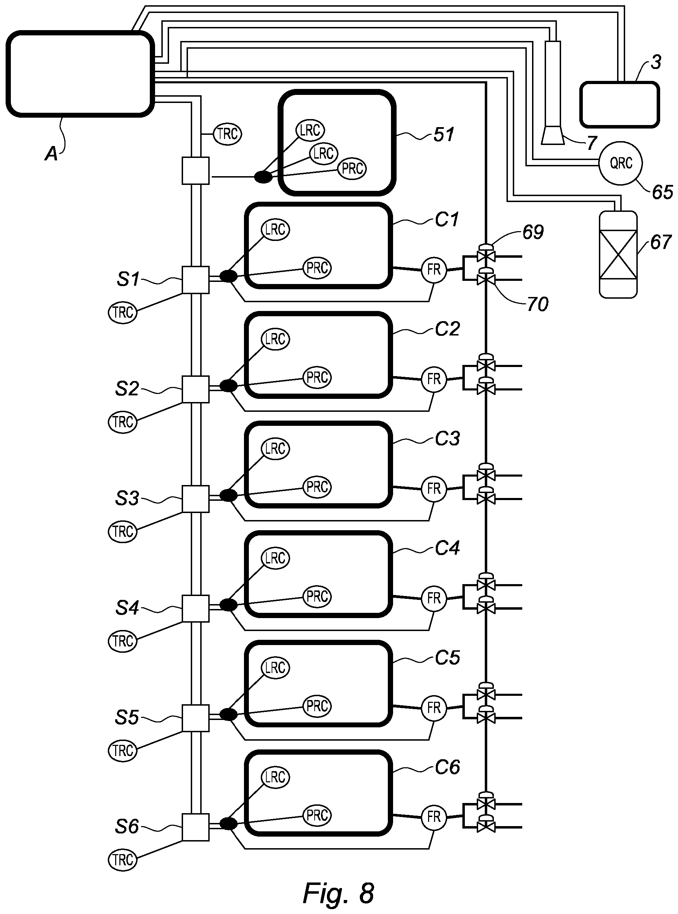

Le schéma représenté à la figure 8 donne des indications quant aux connexions électriques de l'installation selon l'invention, étant noté qu'ici encore tous les organes qui sont représentés à l'extérieur des conteneurs C1,...C6 sont des organes qui se trouvent à résidence, et qui sont disposés notamment sous le niveau de la plateforme 1, comme indiqué par la lettre R de la figure 1.

Comme on peut le voir sur la figure 8, l'armoire électrique A est connectée par des câbles appropriés aux stations d'accueil S1,...S6, lesquelles sont elles-mêmes connectées, par l'intermédiaire de l'interface de connexion 47 représentée à la figure 4a, à des capteurs de niveau de liquide LRC, de pression PRC, voire de température, disposés à l'intérieur de chaque conteneur C1, C6.

Ces stations d'accueil sont en outre connectées à des débitmètres FR permettant de mesurer le débit de gaz traversant les électrovannes 69 et 70 associées à chaque conteneur C.

L'armoire électrique A est par ailleurs connectée électriquement à des capteurs de niveau de liquide LRC et de pression PRC, qui se trouvent dans la cuve de percolât 51.

Cette armoire électrique est en outre connectée aux différents organes permettant le traitement des gaz issus de la fermentation des déchets agricoles, à savoir le cogénérateur 3, la torchère 7, un enregistreur de qualité des gaz 65, une unité de lavage des gaz 67.

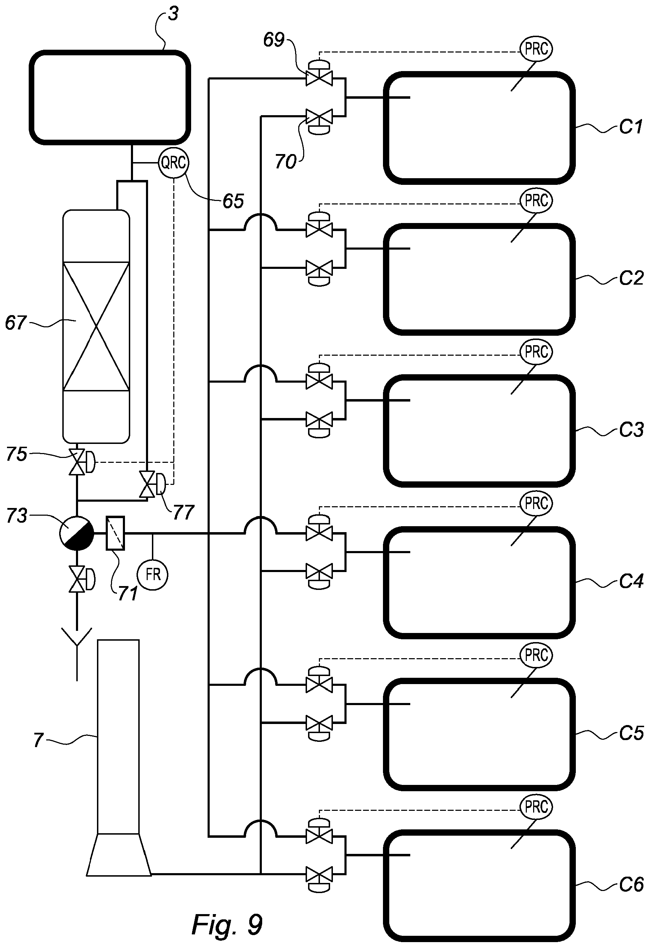

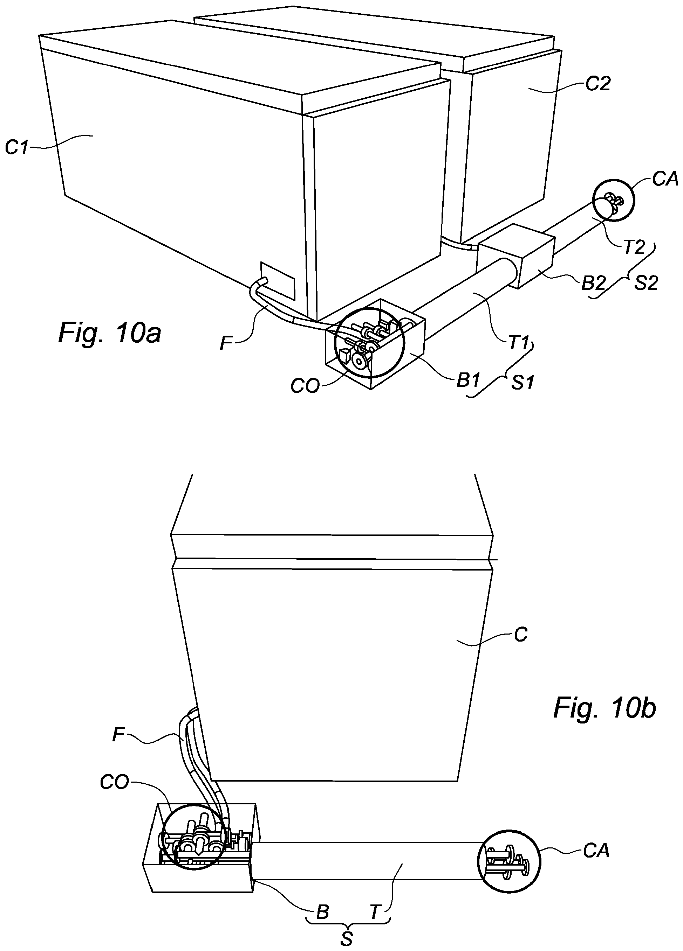

On se reporte à présent à la figure 9, sur laquelle on peut voir le circuit de récupération des gaz issus de la fermentation des déchets d'origine agricole.

Comme précédemment, il faut comprendre que tout ce qui est représenté à l'extérieur des conteneurs C1,...C6 se trouve à résidence sur la plateforme 1 représentée à la figure 1, et correspond aux éléments G, 3, 5, 7 représentés à cette figure.

La connexion des conteneurs C1, ...C6 au réseau de récupération des gaz G se fait par l'intermédiaire du connecteur de récupération des gaz 49 représenté à la figure 4b.

Comme on peut le voir, le réseau fixe de récupération des gaz comprend, pour chaque conteneur C, deux électrovannes 69, 70, susceptibles de conduire les gaz respectivement vers le cogénérateur 3 ou vers la torchère 7, en fonction notamment de la qualité des gaz récupérés.

Lorsque les électrovannes 70 sont fermées et les électrovannes 69 ouvertes, le gaz est acheminé vers un filtre à poussière 71, puis vers une purge à condensat 73.

En fonction des informations délivrées par l'enregistreur de qualité 65 qui commande deux électrovannes 75 et 77, ce gaz est ensuite orienté soit directement vers le cogénérateur 3 afin de produire de l'électricité, ou au préalable vers la colonne de lavage de gaz 67.

Le mode de fonctionnement et les avantages de l'installation selon l'invention résultent directement de la description qui précède.

Dans une exploitation agricole équipée d'une installation telle que représentée à la figure 1, l'agriculteur commence par remplir un ou plusieurs conteneurs mobiles de méthanisation C de déchets d'origine agricole.

Pour ce faire, il extrait chaque conteneur C de son emplacement E en exerçant un effort de traction sur la barre 13 au moyen d'un crochet hydraulique, puis libère les verrous à genouillère.

Ensuite, avec un tracteur à fourche ou bien avec un engin à bras télescopique 25 tel que représenté sur la figure 1, l'agriculteur soulève et retire le couvercle 1 au moyen des deux manchons de préhension 23.

Il transporte ensuite le réceptacle à proximité du lieu de production ou de stockage des matières.

Il vient ensuite remplir l'intérieur du réceptacle 9 des déchets agricoles à traiter.

Lorsque le niveau atteint par ces déchets est quasiment celui des diffuseurs de percolât 29, il rapporte sur la plateforme le réceptacle chargé. Il repose le couvercle 21 puis le referme au moyen des verrous à genouillère disposés à sa périphérie.

Il repousse alors le conteneur C sur son emplacement correspondant E, et vient relier les différents connecteurs 27, 31, 41, 43, 45, 47 à la station d'accueil S correspondante.

Il relie par ailleurs le connecteur de récupération des gaz de fermentation 49 au réseau G.

L'agriculteur attend ensuite le déroulement du cycle de méthanisation, qui peut durer typiquement de 20 à 30 jours.

Pendant ce cycle, le percolât est envoyé périodiquement par les diffuseurs 29 à travers les déchets à traiter, la commande de circulation de ce percolât étant effectuée au moyen des électrovannes 57 et 59.

A noter que des échanges de percolât entre les différents conteneurs C peuvent être obtenus en jouant sur les différentes électrovannes associées, permettant ainsi d'optimiser la digestion pour l'ensemble des conteneurs C.

Pendant la fermentation, on fait également circuler le fluide caloporteur à travers les serpentins 39 de chaque conteneur C, de manière continue ou par intermittence, en fonction de la nature des déchets agricoles à traiter.

A noter que la chambre de tirage 33 et le fond drainant 37 de chaque conteneur C, permettent de récupérer une quantité suffisante de percolât pour pouvoir réaliser les opérations de pompage de ce dernier. Pendant tout le cycle de méthanisation, l'isolation thermique du conteneur C permet de maintenir une température de l'ordre de 37° Celsius, optimale pour la réaction de fermentation anaérobie.

La pression des gaz à l'intérieur de chaque conteneur C est typiquement de l'ordre de 40 mbar, et si pour quelque raison elle devait augmenter, le système de verrou taré par exemple à 100 mbar permet de remplir une fonction de soupape de sécurité.

Bien entendu, tout autre système de soupape de sécurité (disque de sûreté, joint hydraulique, ...) est envisageable en variante.

Les gaz récupérés par le connecteur 49 sont envoyés à l'intérieur du cogénérateur 3, après avoir éventuellement subi une opération de lavage dans la colonne 67 : on peut de la sorte faire tourner un moteur thermique qui entraîne un générateur, produisant de l'électricité pouvant être revendue aux opérateurs du marché.

De manière alternative, le gaz ainsi récupéré, qui comprend essentiellement du méthane, peut être utilisé pour faire fonctionner une chaudière à usage domestique, et/ou permettant de chauffer le fluide caloporteur circulant à l'intérieur des serpentins 39.

On peut aussi envisager, bien que cela soit techniquement un peu plus complexe, d'envoyer le gaz ainsi lavé et récupéré dans le réseau de gaz de ville, après les compressions et purifications nécessaires.

Lorsque le gaz ne présente pas une qualité suffisante, ou bien lorsque l'exploitation agricole n'est pas équipée en conséquence, on peut envoyer ce gaz vers la torchère 7 en vue de réaliser son brûlage.

En fin de cycle, on commence par vidanger le percolât en ouvrant l'électrovanne 59 et en fermant l'électrovanne 57, et on arrête la circulation du fluide caloporteur.

On évacue le gaz exclusivement vers la torchère 7, en ouvrant les électrovannes 70 et en fermant les électrovannes 69, et lorsque la pression de gaz à l'intérieur du conteneur C descend en dessous d'un seuil prédéterminé pendant une durée également prédéterminée, on ferme les vannes manuelles dont sont équipés les connecteurs 27, 31, 41, 43 et on insuffle à l'intérieur du connecteur 45 de l'air sous pression de manière à chasser le reliquat de gaz se trouvant à l'intérieur du conteneur C.

On peut alors ouvrir les verrous qui sont interposés entre le couvercle 21 et le bord supérieur du réceptacle 9, puis enlever ce couvercle 21 au moyen d'un tracteur muni d'une fourche.

On place alors le réceptacle 9 sur une remorque de type « Ampliroll » en exerçant un effort de traction sur la barre 13 au moyen d'un crochet hydraulique solidaire de ladite remorque.

On conduit alors le réceptacle 9 dans un lieu de stockage, ou un champ où l'on souhaite épandre le digestât, c'est-à-dire le produit résultant de l'action des bactéries anaérobies sur les déchets d'origine agricole.

Lorsque l'on est arrivé au site souhaité, on ouvre la porte latérale arrière 15 du réceptacle 9, après avoir ouvert les verrous à crochets 19.

On peut alors soulever la partie avant du réceptacle 9 de manière à vider par l'arrière son contenu.

Comme on peut le comprendre à la lumière de la description qui précède, l'installation selon l'invention est extrêmement intéressante pour une exploitation agricole, car elle est par nature modulaire et, de sorte qu'elle peut coller parfaitement aux besoins de l'exploitant, ainsi qu'à leur évolution.

Plutôt que d 'avoir une seule masse de déchets d'origine agricole en cours de méthanisation, l'installation selon l'invention permet d'avoir différentes masses de moindre volume en cours de traitement, à des stades d'avancement divers, de sorte qu'il peut y avoir en permanence du digestât prêt à l'épandage, ainsi que des conteneurs prêts à recevoir des déchets d'origine agricole.

De plus, comme on aura pu le comprendre à la lumière de ce qui précède, l'installation selon l'invention peut être entièrement automatisée et optimisée, de manière à rendre les réactions de méthanisation le plus efficace possible : on peut en effet régler de manière automatique les cycles de percolation et de chauffage, et mélanger de manière appropriée les percolâts provenant de différents conteneurs.

On peut de la sorte homogénéiser une recette de percolât à l'ensemble des cuves si leur contenu est comparable, ou bien au contraire, particulariser le percolât à chaque cuve, en fonction d'un contenu qui lui est propre.

Les possibilités de prélèvement des mesures en temps réel dans les conteneurs C, ainsi que l'existence d'organes de commande automatisés tels que les électrovannes 57 et 59, permettent d'envisager d'une part le partage en réseau d'informations provenant de plusieurs exploitations différentes, et d'autre part la prise de main à distance sur ces installations par une entité à l'expertise reconnue : on rejoint de la sorte le concept de LIMS (« Laboratory Information Management System » : laboratoire électronique), permettant de mutualiser les connaissances acquises sur des installations qui se trouvent sur des sites différents.

On peut par la suite imaginer un service d'abonnement des différents utilisateurs d'installations selon l'invention, permettant d'accéder en ligne à des données optimisées en fonction des catégories de déchets agricoles à traiter, et des conditions dans lesquelles ce traitement est envisagé.

Bien entendu, la présente invention n'est nullement limitée aux modes de réalisation décrits et représentés, fournis à titre de simples exemples.

## Revendications

1. Module digesteur comprenant un conteneur mobile de méthanisation (C), comprenant un réceptacle (9) de réception de matières organiques telles que des déchets d'origine agricole, un couvercle étanche (21), une porte étanche (15), et un connecteur de récupération des gaz de fermentation (49), le réceptacle (9), le couvercle (21) et la porte (15) étant hermétiques et isolés thermiquement, ledit réceptacle (9) comprenant une pluralité de capteurs, choisis dans le groupe comprenant des capteurs de pression (PRC), des capteurs de température (TRC) et des capteurs de niveau de liquide (LRC), et des moyens de connexion (47) disposés à l'extérieur dudit réceptacle (9), pour relier ces capteurs à un circuit électrique (R), lesdits moyens de connexion (47, 49) étant groupés dans une même zone dudit conteneur (C), facilement accessible.

2. Module (C) selon la revendication 1, conformé pour être placé sur une remorque agricole par exemple de type « ampliroll ».

3. Module (C) selon l'une des revendications 1 ou 2, dans lequel ledit couvercle (21) est rigide.

4. Module (C) selon l'une quelconque des revendications précédentes, dans lequel ledit réceptacle (9) comprend un circuit de diffusion de percolât, comprenant un connecteur extérieur d'entrée de percolât (27), une pluralité de diffuseurs de percolât (29) disposés dans la partie supérieure du réceptacle (9), en communication de fluide avec ledit connecteur d'entrée du percolât, et un connecteur de sortie du percolât (31), en communication de fluide avec une zone de récupération du percolât ayant traversé lesdits déchets agricoles.

5. Module (C) selon la revendication 4, dans lequel ladite zone de récupération du percolât comprend un fond drainant (37) du réceptacle, et une chambre de sous-tirage (33) du percolât, séparée du reste du volume dudit réceptacle (9) par une paroi ajourée (35).

6. Module selon la revendication 4 ou 5 dans lequel lesdits moyens de connexion (27, 31) sont groupés dans une même zone dudit conteneur (C), facilement accessible.

7. Station d'accueil (S) pour module digesteur (C) conforme à l'une quelconque des revendications précédentes, comprenant des connecteurs complémentaires de ceux dudit module (C), et aptes à être reliés à des circuits respectifs de récupération des gaz de fermentation (G), de percolât, de fluide caloporteur, de transmission de données et de courants électriques (R).

8. Station d'accueil (S) selon la revendication 7, comprenant au moins un module préfabriqué comprenant un boîtier (B), un tube (T) connecté à ce boîtier, une pluralité de tronçons de canalisations (CA) situés à l'intérieur de ce tube, comportant chacune un piquage disposé à l'intérieur dudit boîtier et aptes à être connectés aux connecteurs (27, 31, 47, 49) dudit conteneur (C), et une pluralité de vannes motorisées montées à l'extrémité desdits piquages.

9. Installation de méthanisation de matières organiques telles que des déchets d'origine agricole, comprenant :
- une pluralité de modules digesteurs (C1, ... C6) conformes à l'une quelconque des revendications 1 à 6 et de stations d'accueil (S1, ... S6) conformes à la revendication 7, dont les connecteurs complémentaires et moyens de connexion respectifs sont aptes à être accouplés en début de réaction de fermentation, et à être découplés en fin de réaction,
- un circuit de récupération des gaz de fermentation (G), relié d'une part aux connecteurs complémentaires de récupération des gaz des stations d'accueil (S1, ... S6), et d'autre part à au moins une unité de stockage et une unité de traitement, et
- des circuits d'alimentation et de collecte des fluides et données et courants électriques reliant les stations d'accueil.

10. Installation selon la revendication 9, dans laquelle ladite unité de traitement est choisie dans le groupe comprenant une unité de stockage (5), une unité de filtration ou d'épuration du biogaz, une unité de cogénération (3), une unité de lavage de gaz (67), une unité de brûlage (7), une unité de compression, une unité de distribution vers le réseau de gaz de ville, une unité de chargement de véhicule utilisant le biogaz comme carburant.

11. Installation selon la revendication 10, comprenant un circuit de distribution du percolât, comprenant au moins une cuve de stockage de percolât (P), au moins une pompe (55) et une pluralité d'électrovannes (57, 59) interposées entre cette pompe (55) et chaque connecteur d'entrée (27) et de sortie (31) du percolât de chaque station d'accueil (S1, ... S6).

12. Installation selon l'une que quelconque des revendications 9 à 11, dans laquelle lesdites stations d'accueil (S1, ...S6) et lesdits circuits (R, G) forment des modules disponibles en kit de montage.

13. Procédé de commande d'une installation conforme à l'une quelconque des revendications 9 à 12, dans lequel on analyse le déroulement de la réaction de fermentation dans chaque conteneur mobile (C1, ... C6) à partir des données transmises par lesdits capteurs, et on adapte à partir de cette analyse certains réglages de ladite installation de manière à optimiser la réaction de fermentation.

14. Procédé de commande selon la revendication 13, dans lequel on constitue des bases de données permettant de connaître les paramètres de réglage nécessaires à l'optimisation des réactions de fermentation en fonction des caractéristiques des matières à traiter.

## Patentansprüche

1. Fermentermodul, umfassend einen beweglichen Methanisierungscontainer (C), umfassend einen Behälter (9) zur Aufnahme von organischen Stoffen, wie Abfallstoffen mit landwirtschaftlichem Ursprung, eine dichte Abdeckung (21), eine dichte Tür (15) und einen Gärgasgewinnungsanschluss (49), wobei der Behälter (9), die Abdeckung (21) und die Tür (15) luftdicht und wärmeisoliert sind, wobei der Behälter (9) mehrere Sensoren, die aus der Gruppe umfassend Drucksensoren (PRC), Temperatursensoren (TRC) und Flüssigkeitsstandsensoren (LRC) ausgewählt sind, und Anschlussmittel (47) umfasst, die auf der Außenseite des Behälters (9) angeordnet sind, um diese Sensoren mit einem Stromkreis (R) zu verbinden, wobei die Anschlussmittel (47, 49) in derselben Zone des Containers (C), leicht zugänglich, gruppiert sind.

2. Modul (C) nach Anspruch 1, das dazu angepasst ist, auf einem landwirtschaftlichen Anhänger, beispielsweise vom "Ampliroll"-Typ, positioniert zu werden.

3. Modul (C) nach einem der Ansprüche 1 oder 2, wobei die Abdeckung (21) starr ist.

4. Modul (C) nach einem der vorhergehenden Ansprüche, wobei der Behälter (9) einen Perkolatverteilungskreis umfasst, umfassend einen äußeren Perkolateintrittsanschluss (27), mehrere Perkolatverteiler (29), die in dem oberen Teil des Behälters (9) angeordnet sind, in Fluidverbindung mit dem Anschluss zum Einleiten des Perkolats, und einen Perkolataustrittsanschluss (31) in Fluidverbindung mit einer Perkolatgewinnungszone, durch die sich die landwirtschaftlichen Abfallstoffe bewegen.

5. Modul (C) nach Anspruch 4, wobei die Perkolatgewinnungszone einen Drainageboden (37) des Behälters und eine Entnahmekammer (33) für das Perkolat, die von dem Rest des Volumens des Behälters (9) durch eine durchbrochene Wand (35) getrennt ist, umfasst.

6. Modul nach Anspruch 4 oder 5, wobei die Anschlussmittel (27, 31) in derselben Zone des Containers (C), leicht zugänglich, gruppiert sind.

7. Andockstation (S) für ein Fermentermodul (C) nach einem der vorhergehenden Ansprüche, umfassend Anschlüsse, die mit denen des Moduls (C) komplementär sind und dazu geeignet sind, mit jeweiligen Kreisen zur Gewinnung von Gärgasen (G), Perkolat, Kühlmittel, zur Übertragung von Daten und Stromkreisen (R) verbunden zu werden.

8. Andockstation (S) nach Anspruch 7, umfassend mindestens ein vorgefertigtes Modul, umfassend ein Gehäuse (B), ein Rohr (T), das mit diesem Gehäuse verbunden ist, mehrere Kanalisationsabschnitte (CA), die sich im Inneren dieses Rohrs befinden, umfassend jeweils eine Rohrabzweigung, die im Inneren des Gehäuses angeordnet ist, und dazu geeignet, mit den Anschlüssen (27, 31, 47, 49) des Containers (C) verbunden zu werden, und mehrere motorisierte Ventile, die an der Außenseite der Rohrabzweigungen montiert sind.

9. Anlage zur Methanisierung von organischen Stoffen, wie Abfallstoffen mit landwirtschaftlichem Ursprung, umfassend:
- mehrere Fermentermodule (C1, ..., C6) nach einem der Ansprüche 1 bis 6 und Andockstationen (S1, ..., S6) nach Anspruch 7, deren komplementäre Anschlüsse und jeweiligen Anschlussmittel dazu geeignet sind, zum Beginn einer Gärungsreaktion gekoppelt zu werden und zum Ende der Reaktion entkoppelt zu werden,
- einen Gärgasgewinnungskreis (G), der einerseits mit den komplementären Gasgewinnungsanschlüssen der Andockstationen (S1, ..., S6) und andererseits mit mindestens einer Speichereinheit und einer Behandlungseinheit verbunden ist, und
- Zufuhr- und Sammelkreise für Fluide und Daten und Stromkreise, die die Andockstationen verbinden.

10. Anlage nach Anspruch 9, wobei die Behandlungseinheit aus der Gruppe umfassend eine Speichereinheit (5), eine Biogas-Filtrations- oder - Reinigungseinheit, eine Kraft-Wärme-Kopplungseinheit (3), eine Gaswäscheeinheit (67), eine Verbrennungseinheit (7), eine Kompressionseinheit, eine Einheit zur Verteilung in das Stadtgasnetz, eine Einheit zum Laden eines Fahrzeugs, das Biogas als Kraftstoff nutzt, ausgewählt ist.

11. Anlage nach Anspruch 10, umfassend einen Perkolatverteilungskreis, umfassend mindestens einen Perkolatspeichertank (P), mindestens eine Pumpe (55) und mehrere Magnetventile (57, 59), die zwischen dieser Pumpe (55) und jedem Perkolateintrittsanschluss (27) und -austrittsanschluss (31) jeder Andockstation (S1, ..., S6) eingeschoben sind.

12. Anlage nach einem der Ansprüche 9 bis 11, wobei die Andockstationen (S1, ..., S6) und die Kreise (R, G) Module bilden, die in einem Montagesatz verfügbar sind.

13. Verfahren zur Steuerung einer Anlage nach einem der Ansprüche 9 bis 12, wobei der Ablauf der Gärungsreaktion in jedem beweglichen Container (C1, ..., C6) aus Daten, die von den Sensoren übertragen werden, analysiert wird und aus dieser Analyse bestimmte Einstellungen der Anlage angepasst werden, um die Gärungsreaktion zu optimieren.

14. Steuerungsverfahren nach Anspruch 13, wobei Datenbanken erstellt werden, die ermöglichen, die Einstellungsparameter zu kennen, die zur Optimierung von Gärungsreaktionen in Abhängigkeit von Charakteristika von zu behandelnden Stoffen erforderlich sind.

## Claims

1. A digester module comprising a movable methanization container (C), comprising a receptacle (9) for receiving organic matter such as agricultural waste, a sealed cover (21), a sealed door (15), and a fermentation gas recovery connector (49), the receptacle (9), the cover (21) and the door (15) being hermetic and thermally insulated, said receptacle (9) comprising a plurality of sensors, selected from the group comprising pressure sensors (PRC), temperature sensors (TRC) and liquid level sensors (LRC), and connection means (47) which are disposed outside said receptacle (9), for connecting these sensors to an electrical circuit (R), said connection means (47, 49) being grouped in the same area of said container (C), easily accessible.

2. The module (C) according to claim 1, shaped to be placed on an agricultural trailer, for example of the « ampliroll » type.

3. The module (C) according to any of claims 1 or 2, wherein said cover (21) is rigid.

4. The module (C) according to any one of the preceding claims, wherein said receptacle (9) comprises a percolate diffusion circuit, comprising an external percolate inlet connector (27), a plurality of percolate diffusers (29) which are disposed in the upper portion of the receptacle (9), in fluid communication with said percolate inlet connector, and a percolate outlet connector (31), in fluid communication with an area for recovering the percolate which has passed through said agricultural waste.

5. The module (C) according to claim 4, wherein said percolate recovery area comprises a draining bottom (37) of the receptacle, and a percolate sub-drawing chamber (33), separated from the remainder of the volume of said receptacle (9) by an apertured wall (35).

6. The module according to claim 4 or 5, wherein said connection means (27, 31) are grouped in the same area of said container (C), easily accessible.

7. A docking station (S) for a digester module (C) in accordance with any one of the preceding claims, comprising connectors which are complementary to those of said module (C), and capable of being connected to respective circuits of recovery of fermentation gases (G), percolate, heat transfer fluid, data transmission and electric currents (R).

8. The docking station (S) according to claim 7, comprising at least one prefabricated module comprising a casing (B), a tube (T) connected to this casing, a plurality of pipe segments (CA) located inside this tube, each including a tap disposed inside said casing and capable of being connected to the connectors (27, 31, 47, 49) of said container (C), and a plurality of motorized valves which are mounted at the end of said taps.

9. A methanization installation of organic matter such as agricultural waste, comprising:
- a plurality of digester modules (C1, ... C6), in accordance with any one of claims 1 to 6, and of docking stations (S1, ... S6), in accordance with claim 7, whose complementary connectors and respective connection means are capable of being coupled at the beginning of the fermentation reaction, and of being decoupled at the end of the reaction,
- a fermentation gas recovery circuit (G), which is connected, on the one hand, to the complementary gas recovery connectors of the docking stations (S1, ... S6), and on the other hand, to at least one storage unit and one treatment unit, and
- fluid and data supply and collection circuits and electric currents connecting the docking stations.

10. The installation according to claim 9, wherein said treatment unit is selected from the group comprising a storage unit (5), a biogas filtration or purification unit, a cogeneration unit (3), a gas washing unit (67), a burning unit (7), a compression unit, a distribution unit towards the city gas network, a vehicle charging unit using the biogas as fuel.

11. The installation according to claim 10, comprising a percolate distribution circuit, comprising at least one percolate storage tank (P), at least one pump (55) and a plurality of solenoid valves (57, 59) which are interposed between this pump (55) and each inlet (27) and outlet (31) connector of the percolate of each docking station (S1, ...S6).

12. The installation according to any one of claims 9 to 11, wherein said docking stations (S1, ...S6) and said circuits (R, G) form modules which are available as a mounting kit.

13. A method for controlling an installation in accordance with any one of claims 9 to 12, wherein the progress of the fermentation reaction in each movable container (C1, ...C6) is analyzed from the data transmitted by said sensors, and some settings of said installation are adapted from this analysis so as to optimize the fermentation reaction.

14. The control method according to claim 13, wherein databases are established allowing knowing the setting parameters necessary for optimizing the fermentation reactions depending on the characteristics of the matter to be treated.
